# EUROPÄISCHE PATENTSCHRIFT

(11) **EP 2 114 859 B1**
(45) Veröffentlichungstag und Bekanntmachung des Hinweises auf die Patenterteilung: **04.12.2013**
(21) Anmeldenummer: 08708416.6
(22) Anmeldetag: 30.01.2008
(51) Int. Cl.: B01J 37/02, B01J 25/00, B01J 25/02, B01J 35/00, C07C 209/48

(54) **VERFAHREN ZUR HERSTELLUNG VON TRIMETHYLHEXAMETHYLENDIAMIN**
METHOD FOR PRODUCTION OF TRIMETHYLHEXAMETHYLENEDIAMINE
PROCEDE DE PRODUCTION DE TRIMETHYLE-HEXAMETHYLENE-DIAMINE

(30) Priorität: 07.03.2007 DE 102007011484
(43) Veröffentlichungstag der Anmeldung: 11.11.2009
(73) Patentinhaber: Evonik Degussa GmbH, 45128 Essen (DE)
(72) Erfinder: LETTMANN, Christian, 48653 Coesfeld (DE); GRUND, Gerda, 48653 Coesfeld (DE); LIPPE, Juergen, 45891 Gelsenkirchen (DE); KNOOP, Cord, 45721 Haltern Am See (DE)
(86) Internationale Anmeldenummer: PCT/EP2008/051101
(87) Internationale Veröffentlichungsnummer: WO 2008/107236

(56) Entgegenhaltungen:
- WO-A-2005/039766
- DE-C1- 19 540 191
- US-A- 3 862 911

## Beschreibung

Die Erfindung betrifft ein verbessertes Verfahren zur Herstellung von Trimethylhexamethylendiamin, nachfolgend abgekürzt TMD genannt, durch Hydrierung von Trimethylhexamethylendinitril, nachfolgend abgekürzt TMN genannt, in Gegenwart eines geformten Hydrierkatalysators nach Raney.

TMD findet Verwendung als Epoxidharzhärter, als Aminkomponente in Polyamiden sowie als Ausgangskomponente für Trimethylhexamethylendiisocyanat, das seinerseits wiederum Ausgangskomponente für Polyurethansysteme ist. TMD wird industriell bevorzugt durch Hydrierung von TMN hergestellt:

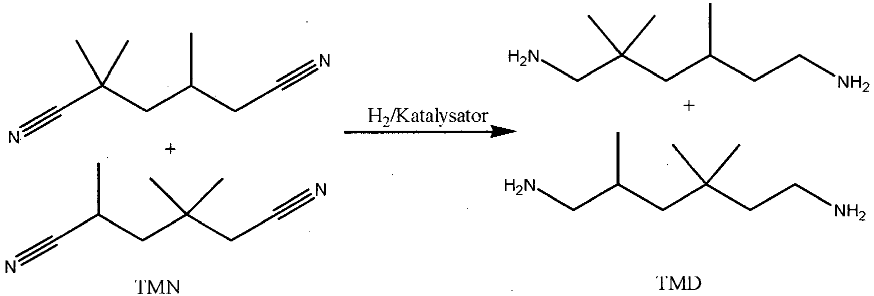

Bedingt durch den Herstellungsprozess des TMN wird in der Hydrierung ein Gemisch aus 2,4,4- und 2,2,4- Trimethylhexamethylendinitril eingesetzt (ca. 60:40). Durch Hydrierung wird ein entsprechendes Isomerengemisch aus 2,4,4- und 2,2.4-Trimethylhexamethylendiamin erhalten.

Wesentliche Nebenreaktionen sind die Bildung von Trimethylcylopentyldiamin und Trimethylazacycloheptan.

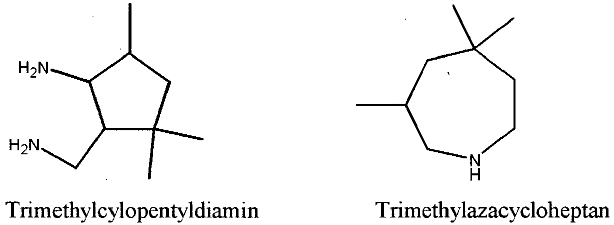

Im Vergleich zur verwandten Umsetzung von Adiponitril zu Hexamethylendiamin ist die Hydrierung vom TMN zu TMD deutlich anspruchsvoller, denn durch die Methylsubstituenten wird die Ringbildung, die zu den beschriebenen Nebenprodukten führt, begünstigt. Dies ist zum einen auf eine erschwerte Hydrierung der Nitrilgruppen durch die sterische Hinderung der Methylsubstituenten zurückzuführen, wodurch die relative Geschwindigkeit der Nebenproduktbildung erhöht wird. Zum anderen stabilisieren die Methylsubstituenten polare Zwischenstufen, die gemäß des Mechanismus der Thorpe-Ziegler-Cyclisierung bei der Ringbildung aus Dinitrilen auftreten:

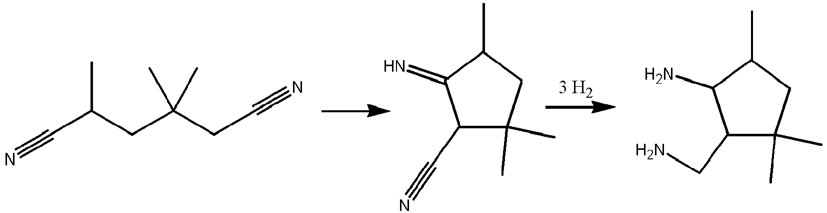

So wird in der DE 15 18 345 erwähnt, dass unter Verwendung eines Raney-Nickel Katalysators bei der Hydrierung von Adiponitril das Zielprodukt Hexamethylendiamin in Ausbeuten größer 90 % erhalten wird, wohingegen unter identischen Reaktionbedingungen bei der Umsetzung von TMN nur eine TMD-Ausbeute von ca. 15 % resultiert. Aktivierte Metallkatalysatoren sind in der chemischen Technik als Raney-Katalysatoren bekannt. Sie werden überwiegend als Pulverkatalysatoren bei einer Großzahl von Hydrierreaktionen eingesetzt. Raney-Katalysatoren werden aus einer Legierung des katalytisch aktiven Metalls und einer in Alkalien löslichen Legierungskomponente hergestellt. Als katalytisch aktive Komponente kommen hauptsächlich Nickel, Kobalt, Kupfer und Eisen zum Einsatz. Als auslaugbarer Legierungsbestandteil findet überwiegend Aluminium Verwendung, aber auch Zink und Silizium sind geeignet. Die so genannte Raney-Legierung wird üblicherweise fein vermahlen, und anschließend wird die auslaugbare Komponente durch Auslaugen mit Alkalien, wie z. B. Natronlauge, ganz oder teilweise entfernt. Pulverkatalysatoren haben den Nachteil, dass sie nur in Batch-Verfahren eingesetzt werden können. Es sind daher verschiedene Verfahren beschrieben worden, die die Herstellung von aktivierten Metall-Festbettkatalysatoren ermöglichen. Solche Festbettkatalysatoren nach Raney sind für die großtechnische Herstellung von TMD besonders geeignet, da sie eine kontinuierliche Prozessführung ermöglichen.

In der DE 43 45 265 und DE 43 35 360 werden geformte Raney-Katalysatoren auf Basis von Ni, Co, Cu und Fe beschrieben, die zur Hydrierung organischer Verbindungen geeignet sind. Der Nachteil der Katalysatoren liegt darin, dass den Katalysatoren Metallpulver als Binder zugesetzt werden muss, und das zugesetzte Metallpulver im Vergleich zum Raney-Metall wenig katalytisch aktiv ist.

In der EP 880 996 wird die Herstellung geformter Raney-Katalysatoren beschrieben, die ohne Zusatz von metallischen Bindemitteln hergestellt werden und zur Hydrierung von Nitrilen eingesetzt werden können. Zur Herstellung dieser Katalysatoren wird eine als Pulver vorliegende Metall-Aluminium-Legierung mit einem hochmolekularen Polymer sowie ggf. Promotoren vermischt und anschließend zu Formkörpern geformt, z. B. durch Extrusion. Die Formkörper werden anschließend bei Temperaturen bis zu 850 °C, kalziniert. Die Temperaturbehandlung führt zur kontrollierten Zersetzung des Polymeren und zur Ausbildung eines Festbettkatalysators mit ausreichender mechanischer Stabilität. Anschließend erfolgt die Aktivierung durch Auslaugung des Aluminiums mittels Natronlauge. Nachteilig an diesem Verfahren ist, dass ein Großteil der eingesetzten Metall-Aluminium-Legierung ungenutzt bleibt, denn die Auslaugung des Aluminiums und somit Aktivierung des Katalysators erfolgt nur in der äußeren Schale des Formkörpers. Der Kern des Katalysators besteht weiterhin aus der eingesetzten Metall-Aluminium-Legierung und ist katalytisch inaktiv, sodass ein erheblicher Teil der relativ teuren Legierungen ungenutzt bleibt und lediglich als Träger für die aktivierte Raney-Metall-Schicht dient.

Eine optimierte Ausnutzung der eingesetzten Raney-Metall-Legierung wird erreicht, wenn Raney-Festbettkatalysatoren zum Einsatz kommen, die in Form von Hohlkörpern vorliegen, wie sie gemäß der Lehre der EP 1 068 900 erhalten werden können. Zur Herstellung der Katalysatoren wird ein Gemisch der erwünschten Legierung, eines organischen Bindemittels und wahlweise eines anorganischen Bindemittels gleichmäßig durch ein Fließbett aus Polystyrolkugeln gesprüht, wo es die Kugeln beschichtet. Die beschichteten Kugeln werden dann bei Temperaturen zwischen 450 und 1000 °C kalziniert, um das Polystyrol herauszubrennen und das Metall zusammenzusintern, um die Hohlform stabiler zu machen. Nach dem Kalzinieren wird der Katalysator durch Natronlauge aktiviert. Die Katalysatoren eignen sich zur Hydrierung, Dehydrierung, Hydratisierung und Isomersierung organischer Verbindungen. Der besondere Vorteil dieser Art von Katalysatoren liegt darin, dass ein großer Teil der eingesetzten Legierung nach der Aktivierung katalytisch aktiv ist, und somit die auf die eingesetzte Masse an Legierung bezogene Aktivität des Katalysators besonders hoch ist. Das Inventar an relativ teurer Legierung im Reaktor kann dadurch minimiert werden.

Nachteilig an den in der EP 1 068 900 beschriebenen Katalysatoren ist der vergleichsweise aufwendige Herstellungsprozess. Eine besonders kritische Phase des Herstellungsprozesses ist der Zeitraum zwischen dem Ausbrennen der Styroporkugeln und der Formierung einer stabilen Schale. Darüber hinaus besitzen die Katalysatoren aufgrund ihrer Hohlkörperstruktur eine geringere Bruchfestigkeit als Katalysatoren, die einen massiven Kern aufweisen. Ferner besitzen die Katalysatoren eine relativ geringe Schüttdichte von nur 0,3 bis 1,3 g/mL, was ihre Anwendung in Festbettreaktoren, die von unten nach oben mit Flüssigkeit durchströmt werden, einschränkt, denn die Katalysatorpartikel können durch das strömende Medium leicht in Bewegung versetzt werden, wodurch die Desaktivierung durch mechanischen Abrieb verstärkt wird.

Die WO2005/039766A beschreibt die Verwendung von IR-, Nah-IR-, sichtbare und UV-Strahlung zur Herstellung von aktivierten Holkörper-Katalysatoren.

Aufgabe der vorliegenden Erfindung ist es, ein Verfahren zur Herstellung von TMD aus TMN zu entwickeln, bei dem Hydrierkatalysatoren nach Raney eingesetzt werden, die möglichst wenig Metalllegierung enthalten, und trotzdem gleiche oder bessere TMD-Ausbeuten erzielt werden als mit den bisher bekannten Verfahren, in denen Hydrierkatalysatoren nach Raney eingesetzt werden.

Überraschend wurde nun gefunden, dass die gestellte Aufgabe durch Verwendung der im Dokument PCT/EP/2005/009656 beschriebenen Katalysatoren gelöst werden kann. Diese Beobachtung ist in sofern überraschend, als das man nicht zwangsläufig davon ausgehen kann, dass bei Einsatz der in der PCT/EP/2005/009656 beschriebenen Katalysatoren im speziellen Fall der Hydrierung des im Vergleich zum Adiponitril sterisch anspruchsvolleren TMN zu TMD die erforderlichen TMD-Ausbeuten erreicht werden.

In der PCT/EP/2005/009656 werden Rancy-Festbettkatalysatoren beschrieben, die durch Aufbringen, insbesondere Aufsprühen einer Raney-Legierung auf einen Träger, wie z. B. Siliziumdioxid oder Aluminiumoxid, erhalten werden können. Durch das Aufbringen, insbesondere Aufsprühen der Legierung auf den Träger werden Formkörper erhalten, bei denen lediglich die äußere Schale aus der Legierung besteht, während der innere Kern der Formkörper aus dem verwendeten Trägermaterial besteht. Durch die Verwendung des Trägermaterials wird der spezifische Einsatz an relativ teurer Legierung minimiert. Die Aktivierung der Formkörper erfolgt in bekannter Weise durch Behandlung mit Säure oder Lauge. Die Vorteile der in der PCT/EP/2005/009656 beschriebenen Katalysatoren gegenüber den Hohlkörpern der EP 1 068 900 sind die weniger aufwendige Produktion und damit verringerte Produktionskosten, eine höhere mechanische Stabilität und eine größere Variabilität der Schüttdichte.

Gegenstand der Erfindung ist ein Verfahren zur Herstellung von Trimethylhexamethylendiamin durch aminierende Hydrierung von Trimethylhexamethylendinitril enthaltenden Gemischen in Gegenwart von mindestens Ammoniak und Wasserstoff, bei dem ein geformter Raney-Typ-Hydrierkatalysator verwendet wird, der nach einem die folgenden Schritte umfassenden Produktionsverfahren hergestellt wurde:
1) Herstellung der Katalysatorvorstufe durch Aufbringen einer pulverförmigen Legierung auf Basis von Kobalt/Nickel/Aluminium auf ein Trägermaterial, wobei als Träger Alumina, Silika, Silika-Alumina, Magnesia, Zinkoxid, Titandioxid, Zirkondioxid, Mischungen dieser Oxide, Keramiken, Formkörper aus Metallen, Glaskugeln, Aktivkohle, Siliziumcarbid, Calciumcarbonat und Bariumsulfat enthalten sind und wobei die pulverförmige Legierung durch Aufsprühen auf das Trägermaterial aufgebracht wird,
2) Trocknung und Kalzinierung der in Schritt 1) erhaltenen Formkörper,
3) Aktivierung der in Schritt 1) oder 2) erhaltenen Formkörper durch Säure und/oder Lauge.

Die erfindungsgemäß zu verwendenden Katalysatoren sind erhältlich nach dem in der PCT/EP/2005/009656 beschriebenen Verfahren.

Die Herstellung der Katalysatorvorstufe erfolgt durch Aufbringen eines oder auch mehrerer Legierungspulver auf ein Trägermaterial. Bei den Trägern kann es sich um die unterschiedlichsten Materialien handeln, z. B. anorganische Oxide wie z. B. Alumina, Silika, Silika-Alumina, Magnesia, Zinkoxid, Titandioxid, Zirkondioxid sowie Mischungen dieser Oxide. Auch andere anorganische Materialien wie Keramiken, Formkörper aus Metallen, Glaskugeln, Aktivkohle, Siliziumcarbid, Calciumcarbonat und Bariumsulfat sind geeignet.

In einer bevorzugten Ausführungsform der Erfindung werden Legierungen auf Basis von Kobalt/Aluminium und/oder Nickel/Aluminium, besonders bevorzugt auf Basis von Kobalt/Nickel/Aluminium, und Träger auf Basis von Alumina, Silika und Alumina-Silika eingesetzt. Es ist vorteilhaft, wenn der Träger ein möglichst geringes Porenvolumen sowie eine relativ inerte Oberfläche aufweist, um am Trägermaterial ablaufende Nebenreaktion zu verhindern.

Die Aufbringung der Legierung auf den Träger erfolgt bevorzugt durch Aufsprühen einer flüssigen Suspension, mindestens enthaltend das oder die Legierungspulver sowie optional eine oder mehrere der folgenden Komponenten: Anorganische Binder (z. B. Ni, Co, Fe, Cu, andere Metallpulver oder anorganische Pulver), organische Binder (z. B. Polyvinylalkohol), Wasser, Promotoren, Porenbildner. Die Partikelgröße der pulverförmigen Legierung liegt im Bereich zwischen 1 und 200 µm. Das Aufbringen der Suspension auf den Träger kann z. B. in einer Trommel oder einer Sprühkammer erfolgen, wobei bei erhöhter Temperatur gearbeitet werden kann, sodass eingebrachte Flüssigkeit, z. B. Wasser, bereits während dieses Präparationsschrittes entfernt wird.

Gegebenenfalls ist eine Vorbehandlung des Trägers notwendig, um die Haftung der aufzubringenden Legierung zu verbessern. Geeignet sind Verfahren, bei denen die Oberfläche des Trägers z. B. durch eine Säurebehandlung oder durch Ätzverfahren angerauht bzw. modifiziert werden. Gegebenfalls kann es von Vorteil sein, den Träger zur Modifizierung seiner Oberflächeneigenschaften mit einem Material vorzubelegen, das als eine Art Bindermittel zwischen Trägermaterial und Legierung wirkt. Als Binder können z. B. anorganische Oxide wie Aluminiumoxid, Titandioxid oder Metallpulver eingesetzt werden. Optional werden die resultierenden Katalysatorvorstufen in einem zusätzlichen Verfahrensschritt weiter getrocknet und kalziniert, bevorzugt bei Temperaturen zwischen 100 und 1200 °C, besonders bevorzugt zwischen 100 und 1000 °C.

Die erfindungsgemäß eingesetzten Katalysatoren können auch aus mehreren Schichten bestehen. Bevorzugt werden die Katalysatorvorstufen dann zwischen den einzelnen Beschichtungsschritten getrocknet, bevorzugt bei Temperaturen zwischen 60 und 150 °C. Die Aktivierung der Katalysatorvorstufe erfolgt durch Auslaugen der löslichen Legierungskomponenten, bevorzugt mit einer wässrigen Mineralbase, wie z. B. Natronlauge. Anschließend wird der aktivierte Katalysator mit Wasser gewaschen.

Das Massenverhältnis von auslaugbarer Legierungskomponente zu Aktivmetallkomponente in der Legierung liegt bevorzugt im Bereich von 20 : 80 bis 80 : 20. Die erfindungsgemäß einzusetzenden Katalysatoren enthalten neben der auslaugbaren Legierungskomponete und der Aktivmetallkomponente bevorzugt weitere Dotierelemente oder Promotoren, ausgewählt aus IIa, IIIb, IVb, Vb, VIb, VIIb, VIII, Ib, IIb, IIIa, IVa und/oder Va, bevorzugt der Reihe der Übergangsmetallgruppen IIIb bis VIIb sowie VIII, einschließlich der seltenen Erden. Auch Hauptgruppenelemente sowie deren Verbindungen, insbesondere die der 1. und 2. Hauptgruppe, sind als Promotoren geeignet. Das Dotieren von Raney-Typ-Katalysatoren ist z. B. in den Dokumenten US 4,153,578, DE 21 01 856, DE 21 00 373 oder DE 20 53 799 beschrieben. Besonders bevorzugte Promotoren sind Magnesium, Chrom, Mangan, Eisen, Kobalt, Vanadium, Tantal, Titan, Cer, Wolfram, Rhenium, Platin, Palladium, Ruthenium, Nickel, Kupfer, Silber, Gold und/oder Molybdän. Ganz besonders bevorzugt sind Magnesium, Chrom und/oder Nickel. Die Promotoren können als Legierungsbestandteil zugesetzt werden und/oder zu einem beliebigen Zeitpunkt während der Präparation, z. B. erst nach dem Aktivierungsschritt. Die Promotoren können sowohl in elementarer Form als auch in Form ihrer Verbindungen zugesetzt werden. Der Anteil an Promotoren im Katalysator beträgt bis zu 20 % bezogen auf das Gesamtgewicht des Katalysators.

Das Schüttgewicht der Katalysatoren kann in einem weiten Bereich zwischen 0,8 und 3 g/mL eingestellt werden und ist insbesondere von der Schüttdichte des Trägermaterials abhängig sowie von dessen Massenanteil am Katalysator, d. h. dem Verhältnis von Trägermasse zur Gesamtmasse des Katalysators.

Das erfindungsgemäße Verfahren zur Herstellung von TMD kann batchweise oder kontinuierlich durchgeführt werden. Bevorzugt wird die Hydrierung kontinuierlich in Festbettreaktoren durchgeführt, die in Riesel- oder Sumpffahrweise betrieben werden können. Geeignete Reaktortypen sind z. B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird. Die Hydrierung erfolgt üblicherweise bei Temperaturen zwischen 20 und 150 °C, bevorzugt zwischen 40 und 130 °C, und Drücken von 0,3 bis 50 MPa, bevorzugt 5 bis 30 MPa. Zur Steuerung des Temperaturverlaufs im Reaktor und insbesondere zur Begrenzung der maximalen Temperatur sind verschiedene, dem Fachmann bekannte Methoden, geeignet. So kann z. B. vollständig ohne zusätzliche Reaktorkühlung gearbeitet werden, wobei das Reaktionsmedium die freiwerdende Energie vollständig aufnimmt und dadurch konvektiv aus dem Reaktor heraus führt. Geeignet sind weiterhin zum Beispiel Hordenreaktoren mit Zwischenkühlung, die Verwendung von Wasserstoffkreisläufen mit Gaskühlung, die Rückführung eines Teils des gekühlten Produktes (Kreislaufreaktor) und die Verwendung von externen Kühlmittelkreisläufen, insbesondere bei Rohrbündelreaktoren.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung von TMN zu TMD an den erfindungsgemäß einzusetzenden Katalysatoren in flüssigem Ammoniak als Lösungsmittel. Pro Mol TMN werden zwischen 1 und 500 Mol, bevorzugt 5 und 200 Mol, besonders bevorzugt zwischen 5 und 100 Mol Ammoniak verwendet.

Obwohl die Hydrierung von TMN zu TMD in Gegenwart von Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol, sowie Ether, besonders THF, MTBE und Dioxan. Der wesentliche Vorteil bei Verwendung eines zusätzlichen Lösungsmittels oder von Lösungsmittelgemischen liegt darin, dass die Hydrierung bei niedrigeren Drücken durchgeführt werden kann, als wenn Ammoniak als alleiniges Lösungsmittel eingesetzt wird.

Das erforderliche Volumen der erfindungsgemäß einzusetzenden Katalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten TMN zu gewährleisten. Üblicherweise liegt der LHSV-Wert bei der Verwendung des bevorzugt einzusetzenden Gemisches aus TMN, Ammoniak und Wasserstoff zwischen 0,5 und 4 m³ TMN/Ammoniak-Mischung pro m³ Katalysator und Stunde, bevorzugt zwischen 1 und 3 m³/(m³ x h).

Das den Hydrierreaktor verlassende Reaktionsgemisch wird in an sich bekannter Weise aufgearbeitet. Diese Aufarbeitung umfasst üblicherweise Abtrennen des Ammoniaks, der Lösungsmittel oder Gemische aus Lösungsmittel und Ammoniak, falls Lösungsmittel vorhanden sind, sowie Isolation des TMD. Das abgetrennte Ammoniak und gegebenenfalls weitere abgetrennte Losungsmittel werden vollständig oder optional nach Ausschleusen eines Teilstroms in den Prozess zurückgeführt.

Außer den zuvor genannten Bestandteilen kann das dem Hydrierreaktor zuzuführende Gemisch zusätzlich höher oder niedriger als TMD siedende Fraktionen enthalten, die bei der destillativen Aufarbeitung des Reaktionsgemisches erhalten werden. Derartige Fraktionen können außer Resten an TMD auch solche Nebenprodukte enthalten, aus denen sich unter Reaktionsbedingungen erneut TMD bildet. Besonders vorteilhaft ist es, nicht vollständig umgesetztes TMN oder Aminonitril enthaltende Fraktionen zurückzuführen.

Es ist bevorzugt aber nicht zwingend erforderlich, dass die erfindungsgemäß einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehrerer Lösungsmittel in Kontakt gebracht. Bevorzugt erfolgt die Konditionierung nach Einbau der Katalysatoren im Hydrierreaktor, sie kann aber auch vor Einbau der Katalysatoren erfolgen. Zur Konditionierung werden zwischen 0,2 und 3, bevorzugt 0,5 und 2 m³ Ammoniak pro m³ Katalysator und Stunde eingesetzt. Üblicherweise wird bei Temperaturen zwischen 20 und 150 °C, bevorzugt 40 bis 130 °C, gearbeitet. Besonders bevorzugt wird eine Temperaturrampe durchfahren, bei der der Katalysator beginnend bei mäßig erhöhter Temperatur, bevorzugt zwischen 20 und 50 °C; langsam bis auf die später für die Hydrierung gewünschte Reaktionstemperatur, bevorzugt 20 bis 150 °C, aufgeheizt wird. Die Konditionierung wird bevorzugt in Gegenwart von Wasserstoff durchgeführt, wobei der Partialdruck des verwendeten Wasserstoffs im Reaktor den Bereich von 0,1 bis 30 MPa, bevorzugt 5 bis 25 MPa, besonders bevorzugt 10 bis 20 MPa umfasst. Die Zeitspanne der Konditionierung ist abhängig von der verwendeten Ammoniakmenge und liegt bevorzugt zwischen 1 und 48 h, besonders bevorzugt zwischen 12 und 24 h.

Unabhängig davon, ob das erfindungsgemäße Verfahren in einer bevorzugten Ausführungsform durchgeführt wird oder nicht, können bei der Umsetzung eines Gemisches aus TMN, Ammoniak, Wasserstoff und gegebenenfalls Lösungsmittel noch eine oder mehrere Hydroxidbasen zugegeben werden. Die Zugabe von Hydroxidbasen kann die Ausbeute an TMD durch Verminderung der Nebenproduktbildung erhöhen. Geeignete Hydroxidbasen sind beispielsweise Alkalihydroxide oder Erdalkalihydroxide. Besonders bevorzugte Hydroxidbasen sind quaternäre Ammoniumhydroxide der allgemeinen Formel (R¹R²R³R⁴N)OH, wobei R¹ bis R⁴ gleich oder unterschiedlich sein können und für aliphatische, cycloaliphatische oder aromatische Reste stehen. Beispiele sind Tetramethyl-, Tetraethyl-, Tetra-n-propyl- und Tetra-n-butylammoniumhydroxid. Geeignete Konzentrationen sind 0,01 bis 100 mmol, bevorzugt 0,05 bis 20 mmol eines Tetraalkylammoniumhydroxids pro Mol TMN.

Es ist auch möglich, bei dem erfindungsgemäßen Verfahren einen oder mehrere Co-Katalysatoren zu verwenden. Geeignete Co-Katalysatoren sind Salze von Kobalt, Nickel, Lanthan, Cer oder Yttrium, bevorzugt Salze von Kobalt und Nickel.

### Beispiele

### Erfindungsgemäßer Katalysator

Es wird eine Beschichtungslösung hergestellt, indem man 776 g einer Co/Al/Cr/Ni-Legierung in 700 g Wasser, das Magensiumnitrat und Polyvinylalkohol enthält, suspendiert. Diese Suspension wird sodann auf 1350 mL Glaskugeln mit einem mittleren Durchmesser von 1.5 bis 2 mm aufgesprüht. Dazu werden die Glaskugeln zunächst in einem nach oben gerichteten Luftstrom suspendiert und auf ca. 80 °C vorgewärmt. Anschließend wird die Suspension aufgesprüht, wobei im Laufe des Aufsprühvorganges eine Temperatur von ca. 90 °C eingestellt wird, um das eingebrachte Wasser zu verdampfen.

Nach dem Beschichten der Glaskugeln mit der zuvor genannten Lösung, werden die Kugeln in aufwärts strömender Luft bei einer Temperatur von ca. 90 °C weiter getrocknet.
In einem zweiten Schritt werden dann 1350 mL der getrockneten, beschichteten Glaskugeln mit einer weiteren Legierungslösung beschichtet.

Es wird eine Beschichtungslösung hergestellt, indem man 675 g einer Co/Al/Cr/Ni-Legierung in 607 g Wasser mit einem Gehalt an 2 Gew.-% Polyvinylalkohol suspendiert.
Die Suspension wird dann unter denselben Bedingungen wie oben beschrieben auf die bereits vorbeschichteten Glaskugeln aufgesprüht.

Nach dem zweiten Beschichtungsschritt werden die beschichteten Glaskugeln in einem Stickstoff/Luftstrom auf 900 °C erwärmt, um den Polyvinylalkohol herauszubrennen und die Legierungsteilchen zusammenzusintern. Die Kugeln werden dann mit Chrom, Nickel und/oder Magnesium in einer 20 Gew.-%igen Natronlauge für 1,5 h bei 90 °C aktiviert.

Die aktivierten Kugeln hatten einen Durchmesser von ca. 3,5 mm und eine Schalendicke von 800 - 900 µm.

### Vergleichskatalysator

Die Herstellung eines Cobalt-Raney-Festbettkatalysators, der in Form einer Hohlkugel vorliegt, erfolgte gemäß der Lehren der Dokumente EP 1 068 900 und EP 1 216 985.
Es wird eine Beschichtungslösung hergestellt, indem man 800 g einer Co/Al/Cr/Ni-Legierung in 1000 mL Wasser, das Magnesiumnitrat und Polyvinylalkohol enthält, suspendiert.

Diese Suspension wird sodann auf 2000 mL Polystyrolkugeln mit einem mittleren Durchmesser von ca. 2 mm aufgesprüht, während diese in einem nach oben gerichteten Luftstrom suspendiert sind. Dazu werden die Styroporkugeln zunächst in einem nach oben gerichteten Luftstrom suspendiert und auf ca. 80 °C vorgewärmt. Anschließend wird die Suspension aufgesprüht, wobei im Laufe des Aufsprühvorganges eine Temperatur von ca. 90 °C eingestellt wird, um das eingebrachte Wasser zu verdampfen.

Nach dem Beschichten der Polystyrolkugeln mit der zuvor genannten Lösung werden die Kugeln in aufwärts strömender Luft bei Temperaturen bis zu 90 °C getrocknet.

In einem zweiten Schritt werden dann 1000 mL dieser getrockneten, beschichteten Polystyrolkugeln mit einer Legierungslösung weiter beschichtet. Die Lösung für die zweite Schicht besteht aus 800 g einer Co/Al/Cr/Ni-Legierung, die in 1000 mL einer wässrigen Lösung aus Magnesiumnitrat und Polyvinylalkohol suspendiert ist.

Die Suspension wird dann unter denselben Bedingungen wie oben beschrieben auf die bereits vorbeschichteten Styroporkugeln aufgesprüht.

Nach dem zweiten Beschichtungsschritt werden die beschichteten Polystyrolkugeln in einem Stickstoff/Luftstrom auf 900 °C erwärmt, um das Polystyrol herauszubrennen und die Legierungsteilchen zusammenzusintern. Die Hohlkugeln werden dann in einer 20 Gew.-%igen Natronlauge für 1,5 h bei 80 °C aktiviert. Die erhaltenen aktivierten Hohlkugeln besitzen Durchmesser im Bereich um 3 mm und eine Manteldicke von ca. 700 µm.

### Hydrierung von TMN mit erfindungsgemäßem Katalysator und Vergleichskatalysator

Die Versuchsapparatur bestand aus drei in Reihe geschalteten, beheizbaren Festbettreaktoren, die jeweils mit ca. 2 L des zu testenden Hydrierkatalysators gefüllt waren. Zur Konditionierung des Katalysators wurden 3 kg Ammoniak bei 100 °C über die Festbetten geleitet. Während der Konditionierung wurde ein Wasserstoffpartialdruck von ca. 100 bar eingestellt. Nach zwölf Stunden wurde die Konditionierung beendet. Im direkten Anschluss an die Konditionierung wurden 6 kg einer auf 50 °C temperierten Lösung von 17 Gew.-% IPN in Ammoniak zugefahren. Der Hydrierreaktor wurde von oben nach unten (Rieselfahrweise) durchströmt. Über ein Regelventil wurde durch Zufuhr von Wasserstoff der Druck im Hydrierreaktor bei 250 bar konstant gehalten. Die Temperatur in den Reaktoren wurde durch externe Beheizung so eingestellt, dass ein Temperaturprofil resultierte, das dem Reaktionsfortschritt bei adiabatischer Betriebsweise entsprach. Die Zusammensetzung des Endproduktes wurde mittels Gaschromatographie ermittelt.

Ein Vergleich der Zusammensetzung der Produkte bei Verwendung des erfindungsgemäßen Katalysators und des Vergleichskatalysators ist in der Tabelle 1 enthalten. Der TMN-Umsatz betrug in allen Versuchen 100 %.

**Tabelle 1**

| **Zusammensetzung des Endproduktes (GC%)** | **Bei Verwendung des erfindungsgemäßen Katalysators** | **Bei Verwendung des Vergleichskatalysators** |
|---|---|---|
| Summe TMD | 96,3 | 95,7 |
| Trimethylcylopentyldiamin | 0,5 | 1,1 |
| Trimethylazacycloheptan | 2,6 | 3 |
| Unbekannte | 0,6 | 0,2 |
| | | |

Die Versuche zeigen, dass die TMD-Ausbeute mit dem erfindungsgemäß zu verwendenden Katalysator höher liegt als bei Verwendung des Vergleichkatalysators.

## Patentansprüche

1. Verfahren zur Herstellung von Trimethylhexamethylendiamin durch aminierende Hydrierung von Trimethylhexamethylendinitril enthaltenden Gemischen in Gegenwart von mindestens Ammoniak und Wasserstoff, bei dem ein geformter Raney-Typ-Hydrierkatalysator verwendet wird, der nach einem die folgenden Schritte umfassenden Produktionsverfahren hergestellt wurde:
1) Herstellung der Katalysatorvorstufe durch Aufbringen einer pulverförmigen Legierung auf Basis von Kobalt/Nickel/Aluminium auf ein Trägermaterial, wobei als Träger Alumina, Silika, Silika-Alumina, Magnesia, Zinkoxid, Titandioxid, Zirkondioxid, Mischungen dieser Oxide, Keramiken, Formkörper aus Metallen, Glaskugeln, Aktivkohle, Siliziumcarbid, Calciumcarbonat und Bariumsulfat enthalten sind und wobei die pulverförmige Legierung durch Aufsprühen auf das Trägermaterial aufgebracht wird,
2) Trocknung und Kalzinierung der in Schritt 1) erhaltenen Formkörper,
3) Aktivierung der in Schritt 1) oder 2) erhaltenen Formkörper durch Säure und/oder Lauge.

2. Verfahren nach mindestens einem der vorherigen Ansprüche,
**dadurch gekennzeichnet,**
**dass** die pulverförmige Legierung anorganische und/oder organische Bindemittel und/oder Promotoren und/oder Säuren und/oder Basen enthält.

3. Verfahren nach mindestens einem der vorherigen Ansprüche,
**dadurch gekennzeichnet,**
**dass** die pulverförmige Legierung Dotiermetalle enthält.

4. Verfahren nach mindestens einem der vorherigen Ansprüche,
**dadurch gekennzeichnet,**
**dass** als Promotoren und/oder Dotiermetalle Verbindungen der folgenden Gruppen des Periodensystems enthalten sind: IIa, IIIb, IVb, Vb, VIb, VIIb, VIII, Ib, IIb, IIIa, IVa und/oder Va.

5. Verfahren nach mindestens einem der vorherigen Ansprüche,
**dadurch gekennzeichnet,**
**dass** Promotoren ausgewählt aus Magnesium, Chrom, Mangan, Eisen, Kobalt, Vanadium, Tantal, Titan, Cer, Wolfram, Rhenium, Platin, Palladium, Ruthenium, Nickel, Kupfer, Silber, Gold und/oder Molybdän enthalten sind.

6. Verfahren nach mindestens einem der vorherigen Ansprüche,
**dadurch gekennzeichnet,**
**dass** Träger auf Basis von Aluminium, Silika und Alumina-Silika enthalten sind.

7. Verfahren nach mindestens einem der vorherigen Ansprüche,
**dadurch gekennzeichnet,**
**dass** als anorganische Binder Metallpulver und/oder organische Pulver enthalten sind.

8. Verfahren nach mindestens einem der vorherigen Ansprüche,
**dadurch gekennzeichnet,**
**dass** als organischer Binder Polyvinylalkohol enthalten ist.

9. Verfahren nach mindestens einem der vorherigen Ansprüche,
**dadurch gekennzeichnet,**
**dass** die Partikelgröße der pulverförmigen Legierung im Bereich zwischen 1 und 200 µm liegt.

10. Verfahren nach mindestens einem der vorherigen Ansprüche,
**dadurch gekennzeichnet,**
**dass** der in Schritt 3) aktivierte Katalysator weiter modifiziert wird.

11. Verfahren nach mindestens einem der vorherigen Ansprüche,
**dadurch gekennzeichnet,**
**dass** die pulverförmige Legierung durch Aufsprühen aus einer flüssigen Suspension aufgebracht wird.

12. Verfahren nach mindestens einem der vorherigen Ansprüche,
**dadurch gekennzeichnet,**
**dass** der Katalysator vor der Hydrierung mit Ammoniak konditioniert wird.

13. Verfahren nach mindestens einem der vorherigen Ansprüche,
**dadurch gekennzeichnet,**
**dass** 4) der in Schritt 3) aktivierte Katalysator weiter modifiziert wird, durch Aufbringen von Metallen und/oder Metallsalzen und/oder Säuren bzw. Basen, und/oder durch Behandlung in reduzierender oder oxidierender Atmosphäre.

14. Verfahren nach mindestens einem der vorherigen Ansprüche,
**dadurch gekennzeichnet,**
**dass** batchweise oder kontinuierlich, ein- oder mehrstufig verfahren wird.

15. Verfahren nach mindestens einem der vorherigen Ansprüche,
**dadurch gekennzeichnet,**
**dass** die Hydrierung kontinuierlich in Festbettreaktoren durchgeführt, die in Riesel- oder Sumpffahrweise betrieben wird.

16. Verfahren nach mindestens einem der vorherigen Ansprüche,
**dadurch gekennzeichnet,**
**dass** die Hydrierung bei Temperaturen zwischen 20 und 150 °C, bevorzugt zwischen 40 und 130 °C, und Drücken von 0,3 bis 50 MPa, bevorzugt 5 bis 30 MPa durchgeführt wird.

## Claims

1. Process for preparing trimethylhexamethylenediamine by aminative hydrogenation of mixtures containing trimethylhexamethylenedinitrile in the presence of at least ammonia and hydrogen, in which a shaped Raney-type hydrogenation catalyst produced by a production process comprising the following steps:
1) production of the catalyst precursor by application of a pulverulent alloy based on cobalt/nickel/aluminium to a support material, with alumina, silica, silica-alumina, magnesia, zinc oxide, titanium dioxide, zirconium dioxide, mixtures of these oxides, ceramics, shaped bodies composed of metals, glass spheres, activated carbon, silicon carbide, calcium carbonate and barium sulphate being present as support and with the pulverulent alloy being applied to the support material by spraying,
2) drying and calcination of the shaped bodies obtained in step 1),
3) activation of the shaped bodies obtained in step 1) or 2) by means of acid and/or alkali, is used.

2. Process according to the preceding claim, **characterized in that**
the pulverulent alloy contains inorganic and/or organic binders and/or promoters and/or acids and/or bases.

3. Process according to at least one of the preceding claims,
**characterized in that**
the pulverulent alloy contains dopant metals.

4. Process according to at least one of the preceding claims,
**characterized in that**
compounds of the following groups of the Periodic Table: IIa, IIIb, IVb, Vb, VIb, VIIb, VIII, Ib, IIb, IIIa, IVa and/or Va are present as promoters and/or dopant metals.

5. Process according to at least one of the preceding claims,
**characterized in that**
promoters selected from among magnesium, chromium, manganese, iron, cobalt, vanadium, tantalum, titanium, cerium, tungsten, rhenium, platinum. palladium, ruthenium, nickel, copper, silver, gold and/or molybdenum are present.

6. Process according to at least one of the preceding claims,
**characterized in that**
supports based on aluminium, silica and aluminasilica are present.

7. Process according to at least one of the preceding claims,
**characterized in that**
metal powders as inorganic binders and/or organic powders are present.

8. Process according to at least one of the preceding claims,
**characterized in that**
polyvinyl alcohol is present as organic binder.

9. Process according to at least one of the preceding claims,
**characterized in that**
the particle size of the pulverulent alloy is in the range from 1 to 200 µm.

10. Process according to at least one of the preceding claims,
**characterized in that**
the catalyst which has been activated in step 3) is modified further.

11. Process according to at least one of the preceding claims,
**characterized in that**
the pulverulent alloy is applied from a liquid suspension by spraying.

12. Process according to at least one of the preceding claims,
**characterized in that** the catalyst is conditioned by means of ammonia before the hydrogenation.

13. Process according to at least one of the preceding claims,
**characterized in that**
4) the catalyst activated in step 3) is modified further, by applying metals and/or metal salts and/or acids or bases, and/or by treatment in reducing or oxidizing atmosphere.

14. Process according to at least one of the preceding claims,
**characterized in that**
it is carried out batchwise or continuously in one or more stages.

15. Process according to any of the preceding claims,
**characterized in that**
the hydrogenation is carried out continuously in fixed-bed reactors which are operated in the downflow mode or upflow mode.

16. Process according to at least one of the preceding claims,
**characterized in that**
the hydrogenation is carried out at temperatures in the range from 20 to 150°C, preferably from 40 to 130°C, and pressures of from 0.3 to 50 MPa, preferably from 5 to 30 MPa.

## Revendications

1. Procédé de fabrication de triméthylhexaméthylènediamine par hydrogénation aminante de mélanges contenant du triméthylhexaméthylène-dinitrile en présence d'au moins de l'ammoniac et de l'hydrogène, selon lequel un catalyseur d'hydrogénation de type Raney façonné est utilisé, qui a été fabriqué par un procédé de production comprenant les étapes suivantes :
1) la fabrication du précurseur de catalyseur par application d'un alliage sous forme de poudre à base de cobalt/nickel/aluminium sur un matériau support, de l'alumine, de la silice, de la silice-alumine, de la magnésie, de l'oxyde de zinc, du dioxyde de zinc, du dioxyde de zirconium, des mélanges de ces oxydes, des céramiques, des corps moulés en métaux, des billes de verre, du charbon actif, du carbure de silicium, du carbonate de calcium et du sulfate de baryum étant contenus en tant que support et l'alliage sous forme de poudre étant appliqué par pulvérisation sur le matériau support,
2) le séchage et la calcination des corps moulés obtenus à l'étape 1),
3) l'activation des corps moulés obtenus à l'étape 1) ou 2) par un acide et/ou une base.

2. Procédé selon la revendication précédente, **caractérisé en ce que** l'alliage sous forme de poudre contient des liants inorganiques et/ou organiques et/ou des promoteurs et/ou des acides et/ou des bases.

3. Procédé selon au moins l'une quelconque des revendications précédentes, **caractérisé en ce que** l'alliage sous forme de poudre contient des métaux dopants.

4. Procédé selon au moins l'une quelconque des revendications précédentes, **caractérisé en ce que** des composés des groupes suivants du tableau périodique sont contenus en tant que promoteurs et/ou métaux dopants : IIa, IIIb, IVb, Vb, VIb, VIIb, VIII, Ib, IIb, IIIa, IVa et/ou Va.

5. Procédé selon au moins l'une quelconque des revendications précédentes, **caractérisé en ce que** des promoteurs choisis parmi le magnésium, le chrome, le manganèse, le fer, le cobalt, le vanadium, le tantale, le titane, le cérium, le tungstène, le rhénium, le platine, le palladium, le ruthénium, le nickel, le cuivre, l'argent, l'or et/ou le molybdène sont contenus.

6. Procédé selon au moins l'une quelconque des revendications précédentes, **caractérisé en ce que** des supports à base d'aluminium, de silice et d'alumine-silice sont contenus.

7. Procédé selon au moins l'une quelconque des revendications précédentes, **caractérisé en ce que** des poudres métalliques et/ou des poudres organiques sont contenues en tant que liants inorganiques.

8. Procédé selon au moins l'une quelconque des revendications précédentes, **caractérisé en ce que** de l'alcool polyvinylique est contenu en tant que liant organique.

9. Procédé selon au moins l'une quelconque des revendications précédentes, **caractérisé en ce que** la taille de particule de l'alliage sous forme de poudre se situe dans la plage comprise entre 1 et 200 µm.

10. Procédé selon au moins l'une quelconque des revendications précédentes, **caractérisé en ce que** le catalyseur activé à l'étape 3) est davantage modifié.

11. Procédé selon au moins l'une quelconque des revendications précédentes, **caractérisé en ce que** l'alliage sous forme de poudre est appliqué par pulvérisation à partir d'une suspension liquide.

12. Procédé selon au moins l'une quelconque des revendications précédentes, **caractérisé en ce que** le catalyseur est conditionné avant l'hydrogénation avec de l'ammoniac.

13. Procédé selon au moins l'une quelconque des revendications précédentes, **caractérisé en ce que** 4) le catalyseur activé à l'étape 3) est davantage modifié par application de métaux et/ou de sels métalliques et/ou d'acides ou de bases et/ou par traitement dans une atmosphère réductrice ou oxydante.

14. Procédé selon au moins l'une quelconque des revendications précédentes, **caractérisé en ce qu'**il est réalisé de manière discontinue ou continue, en une ou plusieurs étapes.

15. Procédé selon au moins l'une quelconque des revendications précédentes, **caractérisé en ce que** l'hydrogénation est réalisée en continu dans des réacteurs à lit fixe exploités en mode de ruissellement ou de fond.

16. Procédé selon au moins l'une quelconque des revendications précédentes, **caractérisé en ce que** l'hydrogénation est réalisée à des températures comprises entre 20 et 150 °C, de préférence entre 40 et 130 °C, et à des pressions allant de 0,3 à 50 MPa, de préférence de 5 à 30 MPa.
